# EUROPEAN PATENT APPLICATION

(11) **EP 4 822 584 A1**
(43) Date of publication of application: **14.10.2026**
(21) Application number: 25890399.6
(22) Date of filing: 15.09.2025
(51) Int. Cl.: G06F 30/25, G06T 17/00, G16C 60/00

(54) **METHOD AND APPARATUS FOR DETERMINING PERCOLATION PERFORMANCE OF CARBON BLACK NETWORK, DEVICE, AND STORAGE MEDIUM**

(30) Priority: 21.02.2025 CN 202510196866
(71) Applicant: Electric Power Research Institute. China Southern Power Grid, Guangzhou, Guangdong 510663 (CN)
(72) Inventor: HOU, Shuai, Guangzhou, Guangdong 510663 (CN); FU, Mingli, Guangzhou, Guangdong 510663 (CN); ZHAN, Yunpeng, Guangzhou, Guangdong 510663 (CN); HUI, Baojun, Guangzhou, Guangdong 510663 (CN); JIA, Lei, Guangzhou, Guangdong 510663 (CN); ZHU, Wenbo, Guangzhou, Guangdong 510663 (CN); FENG, Bin, Guangzhou, Guangdong 510663 (CN); LIU, Jie, Guangzhou, Guangdong 510663 (CN); CHEN, Yun, Guangzhou, Guangdong 510663 (CN)
(74) Representative: Hoffmann Eitle
(86) International application number: PCT/CN2025/121388
(87) International publication number: WO 2026/174776

(57) **Abstract**

The present application provides a method and an apparatus for determining a percolation performance of a carbon black network, a device, and a storage medium. The method comprises: constructing (S202) a percolation model of the carbon black network in a three-dimensional space based on model parameters; gradually inserting (S204) new carbon black particles into the percolation model and obtaining an updated percolation model; searching out (S206) a target conductive path among a plurality of carbon black particles included in the updated percolation model, where the target conductive path passes through a first surface and a second surface in the three-dimensional space; updating (S208) the model parameters, and returning to the step of constructing the percolation model of the carbon black network in the three-dimensional space based on the model parameters, until a iteration termination condition is satisfied; and determining (S210) the percolation performance of the carbon black network based on probabilities of searching out the target conductive path in a plurality of iterations.

## Description

### CROSS-REFERENCE TO RELATED APPLICATIONS

The present application claims priority to Chinese Patent Application No. 202510196866.2, filed on February 21, 2025, and entitled "Method and Apparatus for Determining Percolation Performance of Carbon Black Network, Device, and Storage Medium", the content of which is expressly incorporated herein by reference in its entirety.

### TECHNICAL FIELD

The present application relates to the field of material modeling technology, particularly to a method and an apparatus for determining a percolation performance of a carbon black network, a computer device, a computer-readable storage medium, and a computer program product.

### BACKGROUND

Semiconductive shielding materials are crucial and decisive materials for high-voltage cables, mainly obtained through the melt blending and compounding of base resins, conductive fillers, crosslinking agents, antioxidants, and other processing aids. Conductive fillers are the key components of the semiconductive shielding materials, determining the key performances of the semiconductive shielding materials, such as the electrical, mechanical, thermal, processability, and surface smoothness properties. Conductive carbon black, due to its excellent electrical performance and low cost, has become the primary choice for conductive fillers in the production of semiconductive shielding materials for high-voltage cables. The properties of the conductive carbon black are the key to determining the performance of semiconductive shielding materials. In high-voltage cable shielding materials, the use of conductive carbon black can enhance the electrical conductivity of the material, improve the shielding effect, and enhance the overall performance of the cable. Research shows that the distribution and concentration of the carbon black have a significant impact on the electrical properties of the shielding materials.

A percolation model of the conductive carbon black is of great significance for optimizing material formulations and improving the product performance. Percolation theory is an important tool for studying the electrical properties of the material and can be used to understand how the distribution and connection mode of conductive particles in a matrix affect the overall electrical conductivity of the material. The formation mechanism of carbon black percolation is complex and is influenced by multiple factors, including particle diameter distribution, content ratio, and matrix material property. Accordingly, by constructing a percolation model, the combination mode of the carbon black at different particle diameters and contents can be better simulated, and the percolation performance thereof can be predicted.

In conventional techniques, the construction of a percolation model typically utilizes the cellular automata method to study the percolation phase transition on a two-dimensional regular lattice. Such a modeling approach is relatively simple, but when constructing a three-dimensional percolation model that conforms to the conductive combination property, the accuracy of determining the percolation performance is relatively low.

### SUMMARY

According to the embodiments of the present application, a method and an apparatus for determining a percolation performance of a carbon black network, a computer device, a computer-readable storage medium, and a computer program product are provided, which can improve the accuracy of determining the percolation performance.

A method for determining a percolation performance of a carbon black network is provided, including:
constructing a percolation model of the carbon black network in a three-dimensional space based on model parameters, wherein the percolation model is configured to simulate a distribution of each carbon black particle within the carbon black network in the three-dimensional space;
gradually inserting new carbon black particles into the percolation model and obtaining an updated percolation model;
searching out a target conductive path among a plurality of carbon black particles included in the updated percolation model, wherein the target conductive path passes through a first surface and a second surface in the three-dimensional space, and a height difference between the first surface and the second surface in the three-dimensional space is greater than a difference threshold;
updating the model parameters, and returning to the step of constructing the percolation model of the carbon black network in the three-dimensional space based on the model parameters, until an iteration termination condition is satisfied; and
determining the percolation performance of the carbon black network based on probabilities of searching out the target conductive path in a plurality of iterations.

An apparatus for determining a percolation performance of a carbon black network is provided, including:
a construction module, configured to construct a percolation model of the carbon black network in a three-dimensional space based on model parameters, wherein the percolation model is configured to simulate a distribution of each carbon black particle within the carbon black network in the three-dimensional space;
an update module, configured to gradually insert new carbon black particles into the percolation model to obtain an updated percolation model;
a search module, configured to search out a target conductive path among a plurality of carbon black particles included in the updated percolation model, wherein the target conductive path passes through a first surface and a second surface in the three-dimensional space, and a height difference between the first surface and the second surface in the three-dimensional space is greater than a difference threshold;
the update module being further configured to update the model parameters and return to the step of constructing the percolation model of the carbon black network in the three-dimensional space based on the model parameters until an iteration termination condition is satisfied; and
a determination module, configured to determine the percolation performance of the carbon black network based on probabilities of searching out the target conductive path in a plurality of iterations.

A computer device is provided, including a processor and a memory storing computer-readable instructions. The computer-readable instructions, when executed by the processor, cause the processor to implement the above-mentioned method.

A computer-readable storage medium is provided, on which a computer program is stored. The computer program, when executed by a processor, causes the processor to perform the above-mentioned method.

A computer program product is provided, including a computer program. The computer program, when executed by a processor, causes the processor to perform the above-mentioned method.

The details of one or more embodiments of the present application are provided in the following drawings and descriptions. Other features, purposes, and advantages of the present application will become obvious from the specification, drawings, and claims.

### BRIEF DESCRIPTION OF THE DRAWINGS

To more clearly illustrate the technical solution in the embodiments of the present application or in the conventional technology, accompanying drawings required for the description of the embodiments or the conventional technology are briefly introduced below. Obviously, the drawings described below are merely embodiments of the present application, and those skilled in the art can obtain other drawings based on these disclosed drawings without paying any creative work.
FIG. 1 is an application environment diagram of a method for determining a percolation performance of a carbon black network according to an embodiment.
FIIG. 2 is a flow chart showing a method for determining a percolation performance of a carbon black network according to an embodiment.
FIG. 3 is a schematic diagram illustrating a conductive path according to an embodiment.
FIG. 4 is a schematic diagram illustrating changes in a percolation threshold and a percolation probability of a percolation model according to an embodiment.
FIG. 5 is a schematic diagram illustrating an apparatus for determining a percolation performance of a carbon black network according to an embodiment.
FIG. 6 is an internal structure diagram of a computer device according to an embodiment.

### DETAILED DESCRIPTION OF THE EMBODIMENTS

The technical solution in the embodiments of the present application will be clearly and completely described below in conjunction with the drawings in the embodiments of the present application. Obviously, the described embodiments are merely some embodiments of the present application, rather than all of the embodiments. Based on the embodiments in the present application, all other embodiments obtained by those skilled in the art without any creative work shall fall within the scope of protection of the present application.

To make the purpose, technical solution, and advantages of the present application clearer and more understandable, the present application will be elaborated below in combination with the drawings and embodiments. It should be appreciated that the specific embodiments described herein are merely for the purpose of explaining the present application and are not intended to limit the present application.

Unless otherwise defined, all technical and scientific terms used herein have the same meaning as commonly understood by those skilled in the art to which the present application belongs. The terms used in the description of the present application are only for the purpose of describing specific embodiments and are not intended to limit the present application.

It should be appreciated that the terms "first", "second", etc., used in the present application may be used to describe various components, but these components are not limited by these terms. These terms are only used to distinguish the first component from another component. The expression "multiple" refers to two or more. The expression "at least a part of the component" refers to a part or the whole of the component. It can be appreciated that in the following embodiments, the term "connection" should be understood as "electrical connection", "communication connection", etc., if there exists a transmission of an electrical signal or data between the connected circuits, modules, units, etc. When used herein, the singular form, such as "a", "an", and "the/said" also includes the plural form, unless the context clearly indicates otherwise. It should also be appreciated that the terms "comprising/including" or "having" etc., indicate the existence of the stated features, entireties, steps, operations, components, parts or combinations thereof, but do not exclude the possibility of existence or addition of one or more other features, entireties, steps, operations, components, parts or combinations thereof.

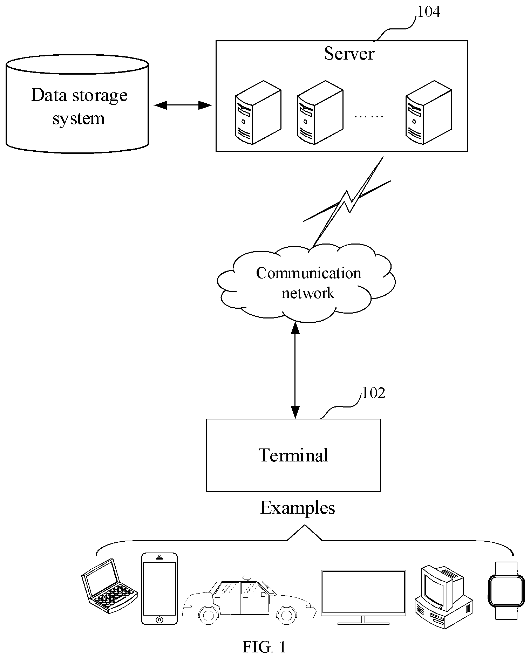

A method for determining a percolation performance of a carbon black network provided in the embodiments of the present application can be applied in the environment as shown in FIG. 1. A terminal 102 is in communication with a server 104 through a network. A data storage system can store data that the server 104 needs to process. The data storage system can be integrated on the server 104 or placed on a cloud or other network servers. The terminal 102 constructs a percolation model of a carbon black network in the three-dimensional space based on model parameters. The percolation model is configured to simulate a distribution of each carbon black particle within the carbon black network in the three-dimensional space. The terminal 102 gradually inserts new carbon black particles into the percolation model to obtain an updated percolation model. The terminal 102 searches out a target conductive path among a plurality of carbon black particles included in the updated percolation model. The target conductive path passes through a first surface and a second surface in the three-dimensional space. A height difference between the first surface and the second surface in the three-dimensional space is greater than a difference threshold. The terminal 102 updates the model parameters and returns to the step of constructing the percolation model of the carbon black network in the three-dimensional space based on the model parameters until an iteration termination condition is satisfied. The terminal 102 determines the percolation performance of the carbon black network based on the probability of searching out the target conductive path in multiple iterations. The terminal 102 may be, but is not limited to, any personal computer, laptop, smartphone, tablet, Internet of Things device, or portable wearable device. The Internet of Things device may be a smart speaker, smart TV, smart air conditioner, smart vehicle equipment, projection equipment, etc. The portable wearable device may be a smart watch, smart bracelet, head-mounted device, etc. The head-mounted device may be a virtual reality (VR) device, an augmented reality (AR) device, smart glasses, etc. The server 104 may be an independent physical server, or a server cluster or distributed system consisting of multiple physical servers, or a cloud server providing a cloud computing service.

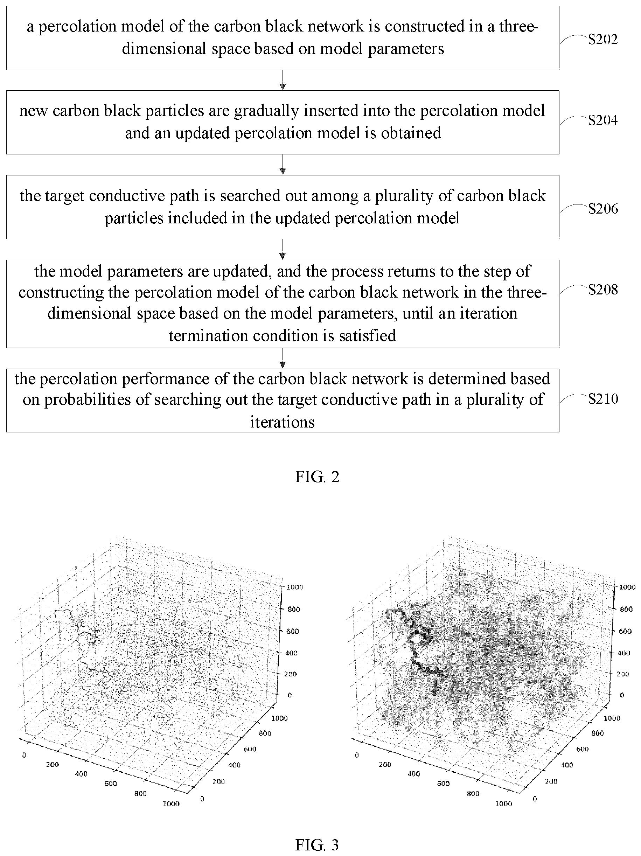

In an exemplary embodiment, as shown in FIG. 2, a method for determining a percolation performance of a carbon black network is provided. The method is applied to the terminal 102 in FIG. 1 as an example for illustration, and the method includes the following steps.

Step S202: a percolation model of the carbon black network is constructed in a three-dimensional space based on model parameters.

The model parameters may refer to an initial condition and a constraint condition for constructing a three-dimensional percolation model, including but not limited to a particle diameter or a particle diameter distribution of a carbon black particle, a spatial size or a geometric shape of a three-dimensional space, a connection distance between particles (such as a critical distance of a tunneling effect), a concentration or a distribution density of carbon black particles, the number of carbon black particle sets consisting of the carbon black particles, a range of the number of carbon black particles included in each carbon black particle set, etc.

The three-dimensional space may be a virtual modeling area simulating the carbon black network, such as a three-dimensional cube or other shapes with clear boundaries, which may provide a spatial range for the distribution of the carbon black particles.

The percolation model of the carbon black network may be a mathematical and physical model configured to describe a distribution and a connection mode of the carbon black particles in the three-dimensional space. The percolation model can be configured to simulate a process of carbon black particles forming a conductive network, and evaluate whether the carbon black network reaches a percolation state.

The percolation model is configured to simulate the distribution of each carbon black particle within the carbon black network in the three-dimensional space.

In a specific implementation, the terminal can distribute the carbon black particles in the three-dimensional space according to the model parameters to form an initial network structure, initialize positions of the carbon black particles according to a particle distribution rule (such as a random distribution, or an aggregation distribution), and set up a connection relationship between the carbon black particles according to a particle connection rule (the distance between two carbon black particles should not be less than a set critical value that may be a tunneling effect distance). By this step, the terminal can obtain the initially constructed percolation model, which provides a basis for the subsequent particle insertion and model update steps.

Step S204: new carbon black particles are gradually inserted into the percolation model, and an updated percolation model is obtained.

Since the gradually inserted carbon black particles can alter the distribution, connection state, and conductive path in the original percolation model, an updated percolation model can be obtained after inserting the new carbon black particles. The updated percolation model can be configured to reflect dynamic changes in the carbon black network structure after the insertion of carbon black particles.

In a specific implementation, the terminal can gradually add carbon black particles based on the original percolation model. At each step, one or more carbon black particles can be inserted, and the target conductive path is searched for among the plurality of carbon black particles in the updated percolation model. When no target conductive path is searched out, the terminal can continue to insert carbon black particles in the next step until a target conductive path is searched out among the plurality of carbon black particles in the updated percolation model.

In a specific implementation, the terminal gradually inserts new carbon black particles into the percolation model, which may refer to randomly generating positions of new carbon black particles in three-dimensional space according to a uniform distribution, or inserting the particles preferentially at efficient connection positions (such as close to the existing conductive groups) according to the distribution state of the existing particles. Each time one new carbon black particle is inserted, the new carbon black particle is checked to see whether it meets the connection condition (such as whether it satisfies the tunneling effect distance), otherwise the position is regenerated.

Step S206: a target conductive path is searched out among a plurality of carbon black particles included in the updated percolation model.

The updated percolation model can be a three-dimensional model which is updated after inserting new carbon black particles into the original percolation model and includes the current distribution and connection relationships of all carbon black particles, reflecting the real-time state of the carbon black network.

The target conductive path passes through a first surface and a second surface in the three-dimensional space. A height difference between the first surface and the second surface in the three-dimensional space is greater than a difference threshold.

The target conductive path may refer to a connected path between carbon black particles in the percolation model formed by a physical contact or the tunneling effect, which indicates the formation of the percolation state.

The first surface and the second surface can be two surfaces in the three-dimensional space between which there exists a significant height difference, for example, an upper surface and a lower surface of the three-dimensional space, or a top portion and a bottom portion of the percolation model. Optionally, the difference threshold may be 80% or 90% of the height of the three-dimensional space.

In an embodiment, the terminal searches for the target conductive path among the plurality of carbon black particles in the updated percolation model, which may refer to using breadth-first search (BFS) or depth-first search (DFS) to search for the target conductive path between the first surface and the second surface. Specifically, the terminal can sequentially traverse the carbon black particles in the three-dimensional space to search for a connected path from a carbon black particle on the first surface to a carbon black particle on the second surface. When the distance between the carbon black particles is less than a preset tunneling effect distance or a physical contact distance, the two carbon black particles are regarded as connected, and it can be determined through recursion and iteration whether the current carbon black particles can connect the first surface and the second surface through the existing connection relationships. During the search for the connected path, path information searched out can be recorded to analyze the influence of different distributions of the carbon black particles on the conductive path. Through search algorithms such as the breadth-first search (BFS) or the depth-first search (DFS), a connected path between an upper surface and a lower surface in a large-scale three-dimensional space can be efficiently searched for, thereby improving the accuracy and stability of the path search.

In a specific implementation, the terminal can perform multiple searches for the target conductive path in the updated percolation model, so that the number of times the target conductive path is successfully searched out in the updated percolation model is recorded, and a percolation probability is obtained through calculation. The percolation probability can be configured to evaluate the percolation performance. The percolation probability may refer to a probability of successfully searching out the target conductive path under a specific distribution condition of carbon black particles, and may be configured to quantify the possibility of the percolation model reaching the percolation state.

Step S208: the model parameters are updated, and the process returns to the step of constructing the percolation model of the carbon black network in the three-dimensional space based on the model parameters, until an iteration termination condition is satisfied.

The model parameters that are updated can be key parameters of the percolation model, such as the particle diameter, particle distribution density, particle content, connection distance, etc. The updated model parameters are configured to reflect the changes in the distribution and the electrical conductivity of the carbon black particles under different conditions, thereby analyzing the impact on the percolation performance.

After the terminal updates the model parameters, the process returns to the step S202 to reconstruct the percolation model of the carbon black network, which is an iterative process that continues until the preset iteration termination condition is satisfied. The iteration termination condition can be configured to determine whether the iteration terminates, such as whether all model parameters such as the particle diameter or particle content are tested or whether the preset number of iterations is reached.

For example, the model parameters are updated, which may refer to changing the particle diameter of the carbon black particle, for instance, from 10 nanometers (nm) to 15 nanometers (nm), or may refer to changing the effective connection distance of the tunneling effect between carbon black particles to reflect different conductive conditions, or may refer to changing the carbon black particle content.

Step S210: the percolation performance of the carbon black network is determined based on probabilities of searching out the target conductive path in a plurality of iterations.

The plurality of iterations may refer to a plurality of processes of updating the model parameters, reconstructing the percolation model, and performing the simulation.

In a specific implementation, in each iteration, the terminal first constructs the percolation model of the carbon black network in the three-dimensional space based on the model parameters, and then gradually inserts new carbon black particles into the percolation model. The gradual insertion of new carbon black particles may refer to increasing the number of carbon black particles successively, rather than adding all the carbon black particles at once. Each time one or more new carbon black particles are inserted, a search for the target conductive path is performed. As new carbon black particles are gradually inserted, the probability of searching out the target conductive path may also change continuously. Each time one carbon black particle is inserted, the search for a path can be performed on the entire updated percolation model, and it is determined whether there exists a connected path from the first surface to the second surface. If the connected path is searched out, "success" is recorded. If no connected path is searched out, the next insertion of a carbon black particle is continued until the target conductive path is searched out among the plurality of carbon black particles in the updated percolation model. Finally, the probability of searching out the target conductive path, i.e., the percolation probability, can be obtained according to the number of times the target conductive path is successfully searched out and the total number of searches in the currently updated percolation model. For example, when only a small number of new carbon black particles need to be inserted to search out the target conductive path, it indicates that the percolation probability of the percolation model constructed based on the model parameters is relatively high. When a large number of new carbon black particles need to be inserted to search out the target conductive path, it indicates that the percolation probability of the percolation model constructed based on the model parameters is relatively low. After the target conductive path is searched out, the model parameters are updated, and the next iteration is performed until the iteration termination condition is satisfied. Subsequently, the percolation performance of the carbon black network is determined based on the probabilities of searching out the target conductive path in the plurality of iterations.

The probability of searching out the target conductive path may also be referred to as the percolation probability. The percolation probability can be configured to reflect the conductive performance of the percolation model with the model parameters. The magnitude of the percolation probability reflects, to some extent, the degree of the conductive network developed by the carbon black particles in the percolation system.

The percolation performance may include the conductive capability and global connectivity characteristics of the carbon black network. The conductive characteristics and percolation behavior of the carbon black network under different conditions can be quantified through the percolation probability.

In a specific implementation, the terminal can summarize the percolation probabilities for multiple iterations, draw relationship curves between different model parameters (such as the particle diameter or particle content) and percolation probabilities, and obtain percolation probability curves under different model parameters, thereby simulating combinations of the carbon black particles under different particle diameters and contents, and determining the impact of different particle diameters and contents on the percolation performance, which helps understand how the distribution and connection mode of the carbon black particles in the matrix affect the overall electrical conductivity of the material.

In the above method for determining the percolation performance of the carbon black network, the terminal constructs the percolation model of the carbon black network in the three-dimensional space based on the model parameters. The percolation model is configured to simulate the distribution of each carbon black particle within the carbon black network in the three-dimensional space. New carbon black particles are gradually inserted into the percolation model to obtain the updated percolation model. The target conductive path is searched for among the plurality of carbon black particles included in the updated percolation model. The target conductive path passes through the first surface and the second surface in the three-dimensional space, and the height difference between the first surface and the second surface in the three-dimensional space is greater than the difference threshold. The model parameters are updated, and the process returns to the step of constructing the percolation model of the carbon black network in the three-dimensional space based on the model parameters, until the iteration termination condition is satisfied. The percolation performance of the carbon black network is determined according to the probabilities of searching out the target conductive path in the plurality of iterations. By gradually inserting carbon black particles and dynamically updating the percolation model, and combining the search for the target conductive path and the statistics of the percolation probabilities, the distribution and connection characteristics of the carbon black particles in the three-dimensional space can be accurately simulated, and a high-precision percolation model can be constructed, so that the accuracy of determining the percolation performance is significantly improved, thereby providing a scientific basis for optimizing the formula of the semiconductive shielding material for the high-voltage cable, and further improving the electrical conductivity, processing performance of the material, and the overall performance of the cable.

In an embodiment, the step of constructing the percolation model of the carbon black network in the three-dimensional space based on the model parameters includes: a preset carbon black particle diameter, a preset number of carbon black particle sets, and a preset number of carbon black particles included in each carbon black particle set in the model parameters are obtained; during the construction of a current carbon black particle set, a plurality of new carbon black particles are generated around the existing carbon black particles in the current carbon black particle set according to a preset particle distance, where the diameter of each of the new carbon black particles conforms to the preset carbon black particle diameter; when the new carbon black particles are located in the three-dimensional space and do not overlap with other carbon black particles in the current carbon black particle set, the new carbon black particles are added into the current carbon black particle set, and the process returns to the step of generating several new carbon black particles around the existing carbon black particles in the current carbon black particle set according to the preset particle distance until the number of carbon black particles included in the current carbon black particle set reaches the preset number of carbon black particles; a next carbon black particle set is continuously constructed, the next carbon black particle set is taken as a current carbon black particle set, and the process returns to the step of generating a plurality of new carbon black particles around the existing carbon black particles in the current carbon black particle set according to the preset particle distance during the construction of the current carbon black particle set, until the preset number of carbon black particle sets are generated; and the percolation model of the carbon black network in the three-dimensional space is constructed based on the plurality of carbon black particle sets.

In practical applications, the distribution mode of carbon black in the matrix resin presents a multi-level and structured feature. A single carbon black particle serves as a basic unit, and carbon black particles are uniformly distributed in the resin through the action of surface active agents or dispersants, forming a primary particle distribution. Subsequently, these particles attract each other through the Van der Waals force to form grape-like collections consisting of several to dozens of primary particles, and these grape-like collections are distributed randomly and with a certain orientation in the matrix, thus forming the distribution of grape-like collections. As the carbon black content increases, the distance between the grape-like collections decreases, which makes it easier to form conductive paths, and these paths construct an effective conductive network in the matrix resin through the direct contact or tunneling effect between the carbon black particles. In addition, an interfacial force between the carbon black particles and the resin also affects the distribution in the matrix. A good interfacial force helps the particles to be more uniformly dispersed and improves the formation of the conductive network, which jointly determines the conductive performance of the carbon black-filled composite material. Accordingly, such a distribution mode can be imitated to construct the carbon black particle sets, and then construct the percolation model of the carbon black network.

The carbon black particle sets can be grape-like carbon black particle sets, and each carbon black particle set can be regarded as a local "grape-like" structure, and an overall carbon black network is formed by combining these sets.

Optionally, the model parameters may include a spatial dimension of the three-dimensional space, the diameter of a carbon black particle, the number of carbon black particles, the number of grape-like structures (the number of carbon black particle sets), the range of the number of spheres (the number of carbon black particle sets) in each grape-like structure, etc. Based on these model parameters, the terminal can generate a plurality of grape-like carbon black particle sets. Specifically, the terminal can take a random position as a starting point and generate the grape-like carbon black particle sets through iterative algorithms to ensure that the newly generated carbon black particles do not overlap with the existing carbon black particles and are within a predetermined range. The terminal divides the generated carbon black particles into first surface carbon black particle sets and second surface carbon black particle sets, and records the corresponding coordinate information. Recursively generated grape-like carbon black particles are uniformly distributed, thereby avoiding the overlap between the particles, and ensuring the rationality and effectiveness of the structure.

In a specific implementation, the terminal can set a starting point in the three-dimensional space and construct the first carbon black particle based on the position of the starting point. The diameter of the first carbon black particle conforms to the preset carbon black particle diameter. According to a random angle and a fixed distance, new carbon black particles are generated from the current carbon black particle. For each time, a plurality of new carbon black particles (for example, 2 to 6) are generated. The newly generated carbon black particles are checked to see whether they are within the three-dimensional space and whether they overlap with an existing carbon black particle. If all the conditions are satisfied, the new carbon black particles can be added to a carbon black particle set. Based on the positions of the new carbon black particles, it is determined whether the new carbon black particles are located on the first or second surface of the three-dimensional space, and the new carbon black particles are added to a corresponding list. If the number of carbon black particles in the current carbon black particle set reaches the preset number of carbon black particles, or the number of attempts to generate a particle reaches 1000 times, the generation of the current carbon black particle set can be ended, and a next carbon black particle set is continuously generated, until the number of carbon black particle sets is equal to the preset number of carbon black particle sets. Consequently, the percolation model of the carbon black network is constructed based on the plurality of carbon black particle sets, and configured to simulate the distribution and electrical conductivity of the carbon black particles in the material.

For example, the dimension of the three-dimensional space can be set to 1000nm × 1000nm × 1000nm, the diameter of the carbon black particle can be adjusted within a range of 10nm to 60nm, the number of carbon black particles in a single grape-like carbon black particle set can be set to range from 15 to 30, and the number of carbon black particle sets can be 200 to 300.

For example, the terminal may record a volume proportion of the generated carbon black particle sets in the entire three-dimensional space to determine a density of the generated structure. Such a calculation helps evaluate the filling effect of carbon black particles in the matrix material.

In the technical solution of the embodiment, the terminal generates carbon black networks with different characteristics (such as particle diameter and density) by adjusting the parameters. Through gradual generation and strict checking, the construction of a high-precision carbon black percolation model in the three-dimensional space is guaranteed. The high-precision carbon black percolation model is adapted to simulate the distribution of carbon black particles in the actual material, and to study the percolation performance or optimize material design.

In an embodiments, the step of gradually inserting the new carbon black particles into the percolation model to obtain the updated percolation model includes: new carbon black particles are gradually inserted into the percolation model; a conductive group to which the new carbon black particle belongs in the percolation model is determined based on a distance between the new carbon black particle and an existing carbon black particle in the percolation model, to obtain an updated conductive group, where the conductive group is a collection of multiple carbon black particles forming a conductive path; and the updated percolation model is obtained based on the updated conductive group.

The conductive group may refer to a collection of multiple carbon black particles connected through the physical contact or tunneling effect. The conductive group is a basis for forming a conductive path in the percolation model.

In a specific implementation, the terminal dynamically inserts new carbon black particles into the existing percolation model, gradually expands or adjusts the conductive network (conductive group) in the model, and determines whether the new particles are connected to a certain conductive group based on the distances between the new particles and the existing particles. If the distance between a new particle and a particle in a certain conductive group is less than a preset connection threshold, the new particle is added to the conductive group. If the new particle simultaneously satisfies connection conditions with multiple conductive groups, these conductive groups are merged into a larger group. If the new particle is not connected to any existing particle, a new independent conductive group is formed. The new carbon black particle is re-grouped with the existing particles to form the updated conductive group, and the structure of the entire percolation model is adjusted based on the updated conductive group to obtain the updated percolation model. The updated percolation model reflects the distribution and connection relationship of the current carbon black particles.

The technical solution of the embodiment reflects the process of carbon black particles from independent distribution to gradually connecting to form a conductive network. The terminal updates the conductive group in real time to ensure that the model accurately describes the connectivity and electrical conductivity of the carbon black particles, which provides a reliable modeling method for studying the relationship among the concentration, distribution, and percolation performance of the carbon black particles. By gradually inserting the carbon black particles, the conductive group and percolation model are dynamically updated, and the precise simulation of the evolution process of the carbon black network is implemented, which is suitable for studying the formation laws of the conductive network and providing an important basis for optimizing material formulations and improving electrical conductivity.

In an embodiment, the step of determining the conductive group to which the new carbon black particle belongs in the percolation model based on the distance between the new carbon black particle and each existing carbon black particle in the percolation model includes: ; based on a distance between the new carbon black particle and each of the existing carbon black particles in the percolation model, determining the existing carbon black particles with the distance less than a preset limit distance as target particles; when each target particle belongs to the same conductive group, determining that the new carbon black particles belong to the same conductive group; when each target particle belongs to one or more different conductive groups, merging the different conductive groups into a new conductive group, and determining that the new carbon black particles belong to the new conductive group.

The preset limit distance serves as a critical distance for determining whether two particles can be connected, and is usually set based on a range of physical contact or tunneling effect.

The target particle can be one of the existing carbon black particles, with the distance between the target particle and the new carbon black particle satisfying the preset limit distance condition.

In a specific implementation, each time the terminal inserts a new carbon black particle, the composition of the conductive group may be adjusted according to the affiliation of the new carbon black particle. For example, an existing conductive group may be expanded, multiple conductive groups may be merged, or a new conductive group may be created (if the new particle is not connected to any target particle).

For example, in the percolation model, when a new carbon black particle is inserted, it is required to determine whether the new carbon black particle forms a new independent conductive group, connects to an existing conductive group, or connects two independent conductive groups. Accordingly, the terminal needs to use the neighborhood relationship in the data structure to determine whether there are other carbon black particles within a given range (within the preset limit distance) around the inserted carbon black particle. For each inserted carbon black particle, a neighboring area is checked to find nearby carbon black particles. Marking values of the nearby carbon black particles are recorded to identify the conductive group to which the nearby carbon black particles belong. The nearby carbon black particles found during the search process are connected. Each inserted carbon black particle is immediately marked, and state information is recorded. As the carbon black particles are inserted gradually, after each insertion, a nearby particle of the new carbon black particle is searched for and the marking value thereof is checked. If carbon black particles with the same marking value are found within the preset limit distance, the new carbon black particle is added to the same conductive group. If carbon black particles with different marking values are found, the two conductive groups are connected and marked with the same marking value. It is possible to randomly select one conductive group and update the marking values of all related carbon black particles to the same one. Accordingly, it is ensured that during the insertion process of particles, the relationship between the particles and the conductive groups can be correctly determined and handled, and the dynamic generation and connection of conductive groups can be implemented.

As an example, setting a preset critical distance for the connection between carbon black particles is crucial in a constructed percolation model. When the spacing between carbon black particles is small, leakage current is generated in the medium, which also enhances the electrical conductivity of the system. Calculations from the formula for the tunneling probability of carriers passing through the barrier show that the tunneling effect decays exponentially with increasing distance, so the preset critical distance can be set to 2 nanometers (nm).

As another example, the terminal can determine the preset limit distance according to the particle diameter of the carbon black particles. Assuming the particle diameter of the carbon black particles is *w*, the limit distance d for connection between the particles can be determined as: *d* = *2r**10⁻³. In the percolation model, the model parameters such as the model specification size, particle diameter, and particle connection condition, etc., are set, particles are gradually inserted into the percolation model, search and connection processes are repeated, and find out other carbon black particles within the preset limit distance of any particle in all 360-degree directions.

In the technical solution of the embodiment, the terminal determines the relationship between the newly inserted carbon black particle and the existing carbon black particles based on the position of the newly inserted carbon black particle, determines the conductive group to which the newly inserted carbon black particle belongs, and merges the conductive groups when necessary, thereby implementing the dynamic adjustment of the conductive network, reflecting the gradual connection and evolution process of the carbon black network, and being able to real-time reflect the impact of the newly inserted particle on the connectivity of the conductive network, supporting the gradual evolution of the carbon black network. By merging the conductive groups, it is ensured that the conductive path of the model is gradually formed and tends to be complete. By filtering the target particles based on the distance, the calculation is only performed on the particles that may be connected, reducing unnecessary operations.

In an embodiment, the step of searching out the target conductive path among the plurality of carbon black particles included in the updated percolation model includes: a maximum conductive group is searched for in the conductive groups included in the updated percolation model; the maximum conductive group includes the largest number of carbon black particles; and when the maximum conductive group passes through the first and second surfaces in the three-dimensional space, a target conductive path is determined from the conductive paths included in the maximum conductive group.

The maximum conductive group includes the largest number of carbon black particles, which represents the most important conductive network in the model. In a specific implementation, all conductive groups in the updated percolation model can be traversed, the number of carbon black particles included in each conductive group is counted, and a conductive group with the largest number of particles is determined as the maximum conductive group.

The maximum conductive group definitely connects the first and second surfaces of the three-dimensional space, that is, the maximum conductive group is required to include the connecting path from the first surface to the second surface. If the maximum conductive group passes through the three-dimensional space, a passing-through path is selected from all conductive paths as the target conductive path. The target conductive path is the most important connecting path in the model, indicating that the model reaches the percolation state.

For example, when the number of inserted carbon black particles in the percolation model increases, the connections between the carbon black particles form conductive groups. When the maximum conductive group passes through the first and second surfaces (such as the upper and lower surfaces) of the system, the terminal can consider that the percolation model reaches the percolation state. When the spatial dimension of the three-dimensional space is a square or a cube, the edge length is equal to 1000, the number of carbon black particles included is denoted as *n,* and the volume fraction is denoted as *V,* then the relationship between V and *n* can be calculated by the following formula: $V = \frac{\left(\frac{4}{3}\right) \times π \times {r}^{3} \times n}{{l}^{3}}$ where *r* represents the particle diameter, and *l* represents the preset limit distance between the carbon black particles.

In the technical solution of the embodiment, the terminal searches for and analyzes the maximum conductive group in the percolation model to search out the target conductive path that can pass through the upper and lower surfaces of the three-dimensional space, thereby implementing an accurate evaluation of the global conductivity of the carbon black network. By determining whether the target conductive path passes through the three-dimensional space, the percolation state can be dynamically analyzed, which provides a scientific basis for studying the percolation performance of the carbon black network.

In an embodiments, the step of determining the percolation performance of the carbon black network based on the probabilities of searching out the target conductive path in the plurality of iterations includes: when the probability of searching out the target conductive path is greater than or equal to a probability threshold, it is determined that the updated percolation model reaches the percolation state, where the percolation state refers to a state where the target conductive path exists in the updated percolation model; and the percolation threshold of the carbon black network is determined according to an average value of the carbon black particle contents when the updated percolation model reaches the percolation state in the plurality of iterations.

In a specific implementation, in each iteration, the terminal calculates the probability of searching out the target conductive path according to the updated percolation model. If the probability of searching out the target conductive path is greater than or equal to the preset probability threshold (such as 50%), it can be determined that the updated percolation model reaches the percolation state. The percolation state means that there exists a conductive path in the carbon black network from the first surface to the second surface of the three-dimensional space.

The terminal records the carbon black particle content when the percolation model reaches the percolation state in each iteration. The carbon black particle content may refer to the proportion of carbon black particles in the model in the total space or the quantity of carbon black particles in the model. The carbon black particle contents corresponding to the percolation state in multiple iterations are summarized, and an average value of the carbon black particle contents is calculated as the percolation threshold of the carbon black network.

The percolation threshold can be configured to represent a difficulty level for the carbon black network to reach the percolation state. A higher percolation threshold indicates that the carbon black network requires a higher particle concentration to form a conductive path, and a lower percolation threshold means that the conductive state can be achieved at a lower concentration.

For example, the percolation threshold obtained through one modeling is accidental. To eliminate accidental errors, the terminal performs multiple iterations and inserts the carbon black particles multiple times in each iteration until the percolation model reaches the percolation state, the carbon black particle content is calculated, the average value of the carbon black particle contents in the iterations is calculated as the percolation threshold, and percolation probability curves under different particle diameters are drawn. The percolation threshold can represent the difficulty level for the carbon black network to reach the percolation state. If the percolation probability of the carbon black network is not less than 50%, it can be considered that the carbon black network reaches the percolation state. The magnitude of the percolation probability reflects the development degree of the conductive network in the percolation system formed by the particles.

Through the technical solution of the embodiment, the terminal dynamically evaluates the electrical conductivity of the carbon black network and precisely determines the percolation state through the setting of a probability threshold. The impact of the distribution of carbon black particles on the electrical conductivity of the carbon black network is quantified through the percolation threshold, thereby improving the accuracy of determining the percolation performance and providing a scientific basis for optimizing the design and production of conductive materials. The method can simulate the combination modes of the carbon black particles under different parameters, such as different particle diameters, different particle contents, and analyze the impact of changes in the particle content and particle diameter on the overall percolation threshold.

In an embodiment, the terminal can generate a three-dimensional structure of the percolation model and a visual image of the conductive paths, and the results are drawn using a graphics library and saved to a specified folder, thereby generating convenient-to-view results and original-sized result images. The probability of searching out the target conductive path in multiple simulations is recorded, the running time of the entire algorithm and running time of each step are calculated and printed, and relevant images are displayed when necessary. The generated three-dimensional structure and path are presented in a graphical form for easy observation and analysis, which provides strong support for further study.

To facilitate the understanding of those skilled in the art, FIG. 3 provides an exemplary schematic diagram illustrating a conductive path.

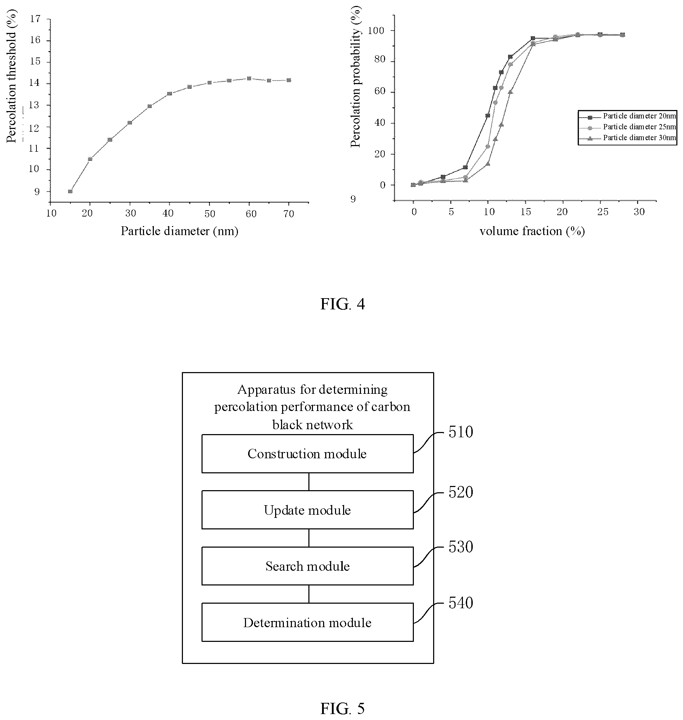

To facilitate the understanding of those skilled in the art, FIG. 4 provides an exemplary schematic diagram illustrating changes in the percolation threshold and percolation probability of a percolation model.

It should be appreciated that although the various steps in the flow charts involved in the embodiments described above are displayed in sequence according to the arrows, these steps are not definitely executed in the order indicated by the arrows. Unless explicitly stated in this document, the execution of these steps is not strictly limited to the sequence indicated by the arrows. These steps may also be executed in any other order. Moreover, at least some of the steps in the flow charts involved in the embodiments described above can include multiple steps or multiple stages, and these steps or stages are not definitely executed at the same time, but may be executed at different moments. These steps or stages are not definitely executed in sequence, but may be executed in turns or alternately with other steps, or at least part of the steps or stages in other steps.

Based on the same inventive concept, in an embodiment of the present application, an apparatus for determining a percolation performance of a carbon black network for implementing the method for determining the percolation performance of the carbon black network is provided. The solution provided by the apparatus for solving the problem is similar to that described in the above method, as for the specific limitations in the following one or more embodiments of the apparatus for determining the percolation performance of the carbon black network, reference can be made to the limitations of the method for determining the percolation performance of the carbon black network described above, and the details will not be repeated here.

In an exemplary embodiment, as shown in FIG. 5, an apparatus for determining a percolation performance of a carbon black network is provided, including a construction module 510, an update module 520, a search module 530, and a determination module 540.

The construction module 510 is configured to construct a percolation model of the carbon black network in a three-dimensional space based on model parameters, where the percolation model is configured to simulate a distribution of each carbon black particle within the carbon black network in the three-dimensional space.

The update module 520 is configured to gradually insert new carbon black particles into the percolation model to obtain an updated percolation model.

The search module 530 is configured to search out a target conductive path among a plurality of carbon black particles included in the updated percolation model, where the target conductive path passes through a first surface and a second surface in the three-dimensional space, and a height difference between the first surface and the second surface in the three-dimensional space is greater than a difference threshold.

The update module 520 is further configured to update the model parameters and return to the step of constructing the percolation model of the carbon black network in the three-dimensional space based on the model parameters until an iteration termination condition is satisfied.

The determination module 540 is configured to determine the percolation performance of the carbon black network based on the probabilities of searching out the target conductive path in a plurality of iterations.

In an embodiment, the update module 520 is specifically configured to: gradually insert new carbon black particles into the percolation model; determine the conductive group to which the new carbon black particle belongs in the permeability model according to the distance between the new carbon black particle and an existing carbon black particle in the permeability model, to obtain an updated conductive group, in which the conductive group is a collection of carbon black particles forming a conductive path; and obtain an updated percolation model based on the updated conductive group.

In an embodiment, the update module 520 is specifically configured to: according to the distance between the new carbon black particle and the existing carbon black particles, determine a plurality of existing carbon black particles each corresponding to the distance less than a preset limit distance as a plurality of target particles; when the plurality of target particles belong to the same conductive group, determine the new carbon black particle to belong to the same conductive group; when the plurality of target particles belong to one or more different conductive groups, merge the different conductive groups into a new conductive group, and determine the new carbon black particle to belong to the new conductive group.

In an embodiment, the search module 530 is specifically configured to: search for a maximum conductive group in the conductive groups included in the updated percolation model, in which the maximum conductive group includes the largest number of carbon black particles; and when the maximum conductive group passes through the first and second surfaces in the three-dimensional space, determine the target conductive path from the conductive paths included in the maximum conductive group.

In an embodiment, the determination module 540 is specifically configured to: when the probability of searching out the target conductive path is greater than or equal to a probability threshold, determine that the updated percolation model reaches a percolation state, in which the percolation state refers to a state where the target conductive path exists updated percolation model; and determine a percolation threshold of the carbon black network according to an average value of the carbon black particle content when the updated percolation model reaches the percolation state during the multiple iterations, in which the percolation threshold is configured to represent a difficulty level for the carbon black network to reach the percolation state.

In an embodiment, the construction module 510 is specifically configured to: obtain a preset carbon black particle diameter, the preset number of carbon black particle sets, and the preset number of carbon black particles included in each carbon black particle set in the model parameters; during the construction of a current carbon black particle set, generate a plurality of new carbon black particles around the existing carbon black particles in the current carbon black particle set according to a preset particle distance, where the diameter of the plurality of new carbon black particles conforms to the preset carbon black particle diameter; when the plurality of new carbon black particles are located in the three-dimensional space and do not overlap with other carbon black particles in the current carbon black particle set, add the plurality of new carbon black particles into the current carbon black particle set, and return to the step of generating a plurality of new carbon black particles around the existing carbon black particles in the current carbon black particle set according to the preset particle distance until the number of carbon black particles included in the current carbon black particle set reaches the preset number of carbon black particles; continue to construct a next carbon black particle set, determine the next carbon black particle set as the current carbon black particle set, and return to the step of generating a plurality of new carbon black particles around the existing carbon black particles in the current carbon black particle set according to the preset particle during the construction of the current carbon black particle set, until the preset number of carbon black particle sets are generated; and construct the percolation model of the carbon black network in the three-dimensional space based on the plurality of carbon black particle sets.

The modules in the above-mentioned apparatus for determining the percolation performance of the carbon black network can all or partially be realized through software, hardware, or combinations thereof. The modules can be embedded in or independent of a processor in a computer device in the form of hardware, or stored in a memory of the computer device in the form of software, so that the processor can invoke and execute the corresponding operations of the modules.

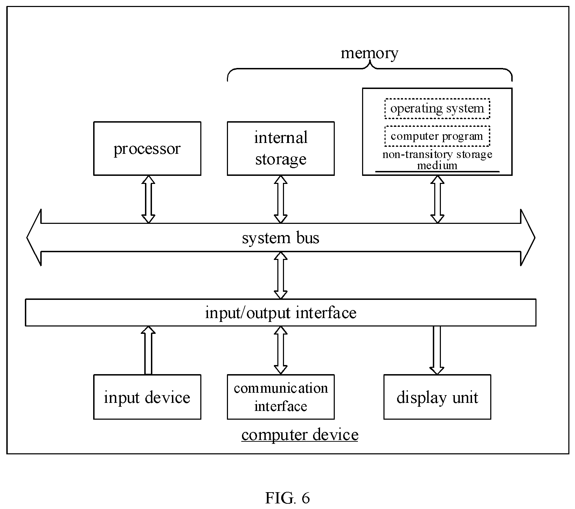

In an exemplary embodiment, a computer device is provided, which can be a terminal, and an internal structure diagram thereof can be as shown in FIG. 6. The computer device includes a processor, a memory, an input/output interface, a communication interface, a display unit, and an input device. The processor, the memory, and the input/output interface are connected through a system bus. The communication interface, the display unit, and the input device are connected to the system bus through the input/output interface. The processor of the computer device is configured to provide computing and control capabilities. The memory of the computer device includes a non-transitory storage medium and an internal storage. The non-transitory storage medium stores an operating system and a computer program. The internal storage provides an environment for the operation of the operating system and the computer program in the non-transitory storage medium. The input/output interface of the computer device is configured to exchange information between the processor and an external device. The communication interface of the computer device is configured to perform wired or wireless communication with an external terminal. The wireless communication can be achieved through WIFI, mobile cellular networks, Near Field Communication (NFC), or other technologies. The computer program, when executed by the processor, implements a method for determining a percolation performance of a carbon black network. The display unit of the computer device is configured to form a visually visible image, which may be a display screen, a projection device, or a virtual reality imaging device. The display screen may be a liquid crystal display screen or an electronic ink display screen. The input device of the computer device may be a touch layer covering the display screen, or a key, a trackball, or a touch pad set on the housing of the computer device, or an external keyboard, touchpad, or mouse, etc.

Those skilled in the art can understand that the structure shown in FIG. 6 is merely a block diagram of a relevant part structure related to the solution of the present application, and does not constitute a limitation on the computer device on which the solution of the present application is applied. The specific computer device may include more or fewer components than those shown in the figure, or combine certain components, or have a different component arrangement.

A computer device includes one or more processors and a memory storing computer-readable instructions. The computer-readable instructions, when executed by the one or more processors, cause the one or more processors to perform the following steps: constructing a percolation model of the carbon black network in a three-dimensional space based on model parameters, wherein the percolation model is configured to simulate a distribution of each carbon black particle within the carbon black network in the three-dimensional space; gradually inserting new carbon black particles into the percolation model and obtaining an updated percolation model; searching out a target conductive path among a plurality of carbon black particles included in the updated percolation model, wherein the target conductive path passes through a first surface and a second surface in the three-dimensional space, and a height difference between the first surface and the second surface in the three-dimensional space is greater than a difference threshold; updating the model parameters, and returning to the step of constructing the percolation model of the carbon black network in the three-dimensional space based on the model parameters, until an iteration termination condition is satisfied; and determining the percolation performance of the carbon black network based on probabilities of searching out the target conductive path in a plurality of iterations.

One or more computer-readable storage media storing computer-readable instructions are provided. The computer-readable instructions, when executed by one or more processors, cause the one or more processors to perform the following steps: constructing a percolation model of the carbon black network in a three-dimensional space based on model parameters, wherein the percolation model is configured to simulate a distribution of each carbon black particle within the carbon black network in the three-dimensional space; gradually inserting new carbon black particles into the percolation model and obtaining an updated percolation model; searching out a target conductive path among a plurality of carbon black particles included in the updated percolation model, wherein the target conductive path passes through a first surface and a second surface in the three-dimensional space, and a height difference between the first surface and the second surface in the three-dimensional space is greater than a difference threshold; updating the model parameters, and returning to the step of constructing the percolation model of the carbon black network in the three-dimensional space based on the model parameters, until an iteration termination condition is satisfied; and determining the percolation performance of the carbon black network based on probabilities of searching out the target conductive path in a plurality of iterations.

A computer program product includes a computer program. The computer program, when executed by a processor, causes the processor to perform the following steps:
constructing a percolation model of the carbon black network in a three-dimensional space based on model parameters, wherein the percolation model is configured to simulate a distribution of each carbon black particle within the carbon black network in the three-dimensional space; gradually inserting new carbon black particles into the percolation model and obtaining an updated percolation model; searching out a target conductive path among a plurality of carbon black particles included in the updated percolation model, wherein the target conductive path passes through a first surface and a second surface in the three-dimensional space, and a height difference between the first surface and the second surface in the three-dimensional space is greater than a difference threshold; updating the model parameters, and returning to the step of constructing the percolation model of the carbon black network in the three-dimensional space based on the model parameters, until an iteration termination condition is satisfied; and determining the percolation performance of the carbon black network based on probabilities of searching out the target conductive path in a plurality of iterations.

An ordinary person skilled in the art can understand that all or part of the processes in the above-mentioned embodiments can be implemented by using a computer program to instruct the related hardware. The computer program may be stored in a non-transitory computer-readable storage medium. When the computer program is executed, the processes in the embodiments of the above-mentioned methods may be included. Any references to memory, database, or other media in the embodiments provided by the present application may include at least one of a non-transitory memory and a transitory memory. The non-transitory memory can include a Read-Only Memory (ROM), a magnetic tape, a floppy disk, a flash memory, an optical storage, a high-density embedded non-transitory memory, a resistive random access memory (ReRAM), a magnetoresistive random access memory (MRAM), a ferroelectric random access memory (FRAM), a phase change memory (PCM), a graphene memory, etc. The transitory memory may include Random Access Memory (RAM) or external high-speed buffer memory, etc. As an illustration rather than limitation, the RAM may take various forms, such as a Static Random Access Memory (SRAM) or a Dynamic Random Access Memory (DRAM), etc. The databases involved in the embodiments provided by the present application may include at least one of a relational database and a non-relational database. The non-relational database may include a blockchain-based distributed database, etc., not limited to these. The processors involved in the embodiments provided by the present application may be general-purpose processors, central processors, graphics processors, digital signal processors, programmable logic devices, quantum computing data processing logic devices, Artificial Intelligence (AI) processors, etc., not limited to these.

The technical features in the above embodiments may be combined in any way. In order to make the description concise, all possible combinations of the technical features in the above embodiments are not described. However, as long as there is no contradiction in the combinations of these technical features, these combinations should be considered to be within the scope of the present application.

The above-described embodiments only express several implementation modes of the present application, and the descriptions are relatively specific and detailed, but should not be construed as limiting the scope of the present application. It should be noted that those of ordinary skill in the art can make several transformations and improvements without departing from the concept of the present application, and these all fall within the protection scope of the present application. Therefore, the protection scope of the present application should be subject to the appended claims.

## Claims

1. A method for determining a percolation performance of a carbon black network, comprising:
constructing a percolation model of the carbon black network in a three-dimensional space based on model parameters, wherein the percolation model is configured to simulate a distribution of each carbon black particle within the carbon black network in the three-dimensional space;
gradually inserting new carbon black particles into the percolation model and obtaining an updated percolation model;
searching out a target conductive path among a plurality of carbon black particles included in the updated percolation model, wherein the target conductive path passes through a first surface and a second surface in the three-dimensional space, and a height difference between the first surface and the second surface in the three-dimensional space is greater than a difference threshold;
updating the model parameters, and returning to the step of constructing the percolation model of the carbon black network in the three-dimensional space based on the model parameters, until an iteration termination condition is satisfied; and
determining the percolation performance of the carbon black network based on probabilities of searching out the target conductive path in a plurality of iterations.

2. The method according to claim 1, wherein gradually inserting new carbon black particles into the percolation model and obtaining the updated percolation model comprises:
gradually inserting the new carbon black particle into the percolation model;
determining a conductive group to which the new carbon black particle belongs in the percolation model based on a distance between the new carbon black particle and each existing carbon black particle in the percolation model, to obtain an updated conductive group, wherein the conductive group is a collection of multiple carbon black particles forming a conductive path; and
obtaining the updated percolation model based on the updated conductive group.

3. The method according to claim 2, wherein determining the conductive group to which the new carbon black particle belongs in the percolation model based on the distance between the new carbon black particle and each existing carbon black particle in the percolation model comprises:
based on a distance between the new carbon black particle and each of the existing carbon black particles in the percolation model, determining the existing carbon black particles with the distance less than a preset limit distance as target particles;
when the target particles belong to a same conductive group, determining that the new carbon black particle belongs to the same conductive group; and
when the target particles belong to one or more different conductive groups, merging the different conductive groups into a new conductive group, and determining that the new carbon black particle belongs to the new conductive group.

4. The method according to claim 2, wherein searching out the target conductive path among the plurality of carbon black particles included in the updated percolation model comprises:
searching for a maximum conductive group in conductive groups included in the updated percolation model, wherein the maximum conductive group comprises the largest number of carbon black particles; and
when the maximum conductive group passes through the first surface and the second surface in the three-dimensional space, determining the target conductive path from conductive paths included in the maximum conductive group.

5. The method according to claim 1, wherein determining the percolation performance of the carbon black network based on the probabilities of searching out the target conductive path in the plurality of iterations comprises:
when the probability of searching out the target conductive path is greater than or equal to a probability threshold, determining that the updated percolation model reaches a percolation state, wherein the percolation state refers to a state where the target conductive path exists in the updated percolation model; and
determining a percolation threshold of the carbon black network according to an average value of carbon black particle contents when the updated percolation model reaches the percolation state in the plurality of iterations, wherein the percolation threshold is configured to represent a difficulty level for the carbon black network to reach the percolation state.

6. The method according to claim 1, wherein constructing the percolation model of the carbon black network in the three-dimensional space based on the model parameters comprises:
obtaining, from the model parameters, a preset carbon black particle diameter, a preset number of carbon black particle sets, and a preset number of carbon black particles included in each carbon black particle set;
during construction of a current carbon black particle set, generating a plurality of new carbon black particles around existing carbon black particles in the current carbon black particle set according to a preset particle distance, wherein a diameter of each of the new carbon black particles conforms to the preset carbon black particle diameter;
when the plurality of new carbon black particles are located in the three-dimensional space and do not overlap with other carbon black particles in the current carbon black particle set, adding the plurality of new carbon black particles into the current carbon black particle set and returning to the step of generating a plurality of new carbon black particles around the existing carbon black particles in the current carbon black particle set according to the preset particle distance, until the number of carbon black particles included in the current carbon black particle set reaches the preset number of carbon black particles;
continuing to construct a next carbon black particle set, determining the next carbon black particle set as the current carbon black particle set, and returning to the step of during construction of a current carbon black particle set, generating a plurality of new carbon black particles around the existing carbon black particles in the current carbon black particle set according to the preset particle distance, until the preset number of carbon black particle sets are generated; and
constructing the percolation model of the carbon black network in the three-dimensional space based on the plurality of carbon black particle sets.

7. An apparatus for determining a percolation performance of a carbon black network, comprising:
a construction module, configured to construct a percolation model of the carbon black network in a three-dimensional space based on model parameters, wherein the percolation model is configured to simulate a distribution of each carbon black particle within the carbon black network in the three-dimensional space;
an update module, configured to gradually insert new carbon black particles into the percolation model to obtain an updated percolation model;
a search module, configured to search out a target conductive path among a plurality of carbon black particles included in the updated percolation model, wherein the target conductive path passes through a first surface and a second surface in the three-dimensional space, and a height difference between the first surface and the second surface in the three-dimensional space is greater than a difference threshold;
the update module being further configured to update the model parameters and return to the step of constructing the percolation model of the carbon black network in the three-dimensional space based on the model parameters until an iteration termination condition is satisfied; and
a determination module, configured to determine the percolation performance of the carbon black network based on probabilities of searching out the target conductive path in a plurality of iterations.

8. A computer device comprising one or more processors and a memory storing computer-readable instructions, wherein the computer-readable instructions, when executed by the one or more processors, cause the one or more processors to perform following steps:
constructing a percolation model of the carbon black network in a three-dimensional space based on model parameters, wherein the percolation model is configured to simulate a distribution of each carbon black particle within the carbon black network in the three-dimensional space;
gradually inserting new carbon black particles into the percolation model and obtaining an updated percolation model;
searching out a target conductive path among a plurality of carbon black particles included in the updated percolation model, wherein the target conductive path passes through a first surface and a second surface in the three-dimensional space, and a height difference between the first surface and the second surface in the three-dimensional space is greater than a difference threshold;
updating the model parameters, and returning to the step of constructing the percolation model of the carbon black network in the three-dimensional space based on the model parameters, until an iteration termination condition is satisfied; and
determining the percolation performance of the carbon black network based on probabilities of searching out the target conductive path in a plurality of iterations.

9. The computer device according to claim 8, wherein the processors, when executing the computer-readable instructions, are further configured to perform following steps:
gradually inserting the new carbon black particle into the percolation model;
determining a conductive group to which the new carbon black particle belongs in the percolation model based on a distance between the new carbon black particle and each existing carbon black particle in the percolation model, to obtain an updated conductive group, wherein the conductive group is a collection of multiple carbon black particles forming a conductive path; and
obtaining the updated percolation model based on the updated conductive group.

10. The computer device according to claim 9, wherein the processors, when executing the computer-readable instructions, are further configured to perform following steps:
based on a distance between the new carbon black particle and each of the existing carbon black particles in the percolation model, determining the existing carbon black particles with the distance less than a preset limit distance as target particles;
when the target particles belong to a same conductive group, determining that the new carbon black particle belongs to the same conductive group; and
when the target particles belong to one or more different conductive groups, merging the different conductive groups into a new conductive group, and determining that the new carbon black particle belongs to the new conductive group.

11. The computer device according to claim 9, wherein the processors, when executing the computer-readable instructions, are further configured to perform following steps:
searching for a maximum conductive group in conductive groups included in the updated percolation model, wherein the maximum conductive group comprises the largest number of carbon black particles; and
when the maximum conductive group passes through the first surface and the second surface in the three-dimensional space, determining the target conductive path from conductive paths included in the maximum conductive group.

12. The computer device according to claim 8, wherein the processors, when executing the computer-readable instructions, are further configured to perform following steps:
when the probability of searching out the target conductive path is greater than or equal to a probability threshold, determining that the updated percolation model reaches a percolation state, wherein the percolation state refers to a state where the target conductive path exists in the updated percolation model; and
determining a percolation threshold of the carbon black network according to an average value of carbon black particle contents when the updated percolation model reaches the percolation state in the plurality of iterations, wherein the percolation threshold is configured to represent a difficulty level for the carbon black network to reach the percolation state.

13. The computer device according to claim 8, wherein the processors, when executing the computer-readable instructions, are further configured to perform following steps:
obtaining, from the model parameters, a preset carbon black particle diameter, a preset number of carbon black particle sets, and a preset number of carbon black particles included in each carbon black particle set;
during construction of a current carbon black particle set, generating a plurality of new carbon black particles around existing carbon black particles in the current carbon black particle set according to a preset particle distance, wherein a diameter of each of the new carbon black particles conforms to the preset carbon black particle diameter;
when the plurality of new carbon black particles are located in the three-dimensional space and do not overlap with other carbon black particles in the current carbon black particle set, adding the plurality of new carbon black particles into the current carbon black particle set and returning to the step of generating a plurality of new carbon black particles around the existing carbon black particles in the current carbon black particle set according to the preset particle distance, until the number of carbon black particles included in the current carbon black particle set reaches the preset number of carbon black particles;
continuing to construct a next carbon black particle set, determining the next carbon black particle set as the current carbon black particle set, and returning to the step of during construction of a current carbon black particle set, generating a plurality of new carbon black particles around the existing carbon black particles in the current carbon black particle set according to the preset particle distance, until the preset number of carbon black particle sets are generated; and
constructing the percolation model of the carbon black network in the three-dimensional space based on the plurality of carbon black particle sets.

14. One or more computer-readable storage media, on which computer-readable instructions are stored, wherein the computer-readable instructions, when executed by one or more processors, cause the one or more processors to perform following steps:
constructing a percolation model of the carbon black network in a three-dimensional space based on model parameters, wherein the percolation model is configured to simulate a distribution of each carbon black particle within the carbon black network in the three-dimensional space;
gradually inserting new carbon black particles into the percolation model and obtaining an updated percolation model;
searching out a target conductive path among a plurality of carbon black particles included in the updated percolation model, wherein the target conductive path passes through a first surface and a second surface in the three-dimensional space, and a height difference between the first surface and the second surface in the three-dimensional space is greater than a difference threshold;
updating the model parameters, and returning to the step of constructing the percolation model of the carbon black network in the three-dimensional space based on the model parameters, until an iteration termination condition is satisfied; and
determining the percolation performance of the carbon black network based on probabilities of searching out the target conductive path in a plurality of iterations.

15. The computer-readable storage media according to claim 14, wherein the processors, when executing the computer-readable instructions, are further configured to perform following steps:
gradually inserting the new carbon black particle into the percolation model;
determining a conductive group to which the new carbon black particle belongs in the percolation model based on a distance between the new carbon black particle and each existing carbon black particle in the percolation model, to obtain an updated conductive group, wherein the conductive group is a collection of multiple carbon black particles forming a conductive path; and
obtaining the updated percolation model based on the updated conductive group.

16. The computer-readable storage media according to claim 15, wherein the processors, when executing the computer-readable instructions, are further configured to perform following steps:
based on a distance between the new carbon black particle and each of the existing carbon black particles in the percolation model, determining the existing carbon black particles with the distance less than a preset limit distance as target particles;
when the target particles belong to a same conductive group, determining that the new carbon black particle belongs to the same conductive group; and
when the target particles belong to one or more different conductive groups, merging the different conductive groups into a new conductive group, and determining that the new carbon black particle belongs to the new conductive group.

17. The computer-readable storage media according to claim 15, wherein the processors, when executing the computer-readable instructions, are further configured to perform following steps:
searching for a maximum conductive group in conductive groups included in the updated percolation model, wherein the maximum conductive group comprises the largest number of carbon black particles; and
when the maximum conductive group passes through the first surface and the second surface in the three-dimensional space, determining the target conductive path from conductive paths included in the maximum conductive group.

18. The computer-readable storage media according to claim 14, wherein the processors, when executing the computer-readable instructions, are further configured to perform following steps:
when the probability of searching out the target conductive path is greater than or equal to a probability threshold, determining that the updated percolation model reaches a percolation state, wherein the percolation state refers to a state where the target conductive path exists in the updated percolation model; and
determining a percolation threshold of the carbon black network according to an average value of carbon black particle contents when the updated percolation model reaches the percolation state in the plurality of iterations, wherein the percolation threshold is configured to represent a difficulty level for the carbon black network to reach the percolation state.

19. The computer-readable storage media according to claim 14, wherein the processors, when executing the computer-readable instructions, are further configured to perform following steps:
obtaining, from the model parameters, a preset carbon black particle diameter, a preset number of carbon black particle sets, and a preset number of carbon black particles included in each carbon black particle set;
during construction of a current carbon black particle set, generating a plurality of new carbon black particles around existing carbon black particles in the current carbon black particle set according to a preset particle distance, wherein a diameter of each of the new carbon black particles conforms to the preset carbon black particle diameter;
when the plurality of new carbon black particles are located in the three-dimensional space and do not overlap with other carbon black particles in the current carbon black particle set, adding the plurality of new carbon black particles into the current carbon black particle set and returning to the step of generating a plurality of new carbon black particles around the existing carbon black particles in the current carbon black particle set according to the preset particle distance, until the number of carbon black particles included in the current carbon black particle set reaches the preset number of carbon black particles;
continuing to construct a next carbon black particle set, determining the next carbon black particle set as the current carbon black particle set, and returning to the step of during construction of a current carbon black particle set, generating a plurality of new carbon black particles around the existing carbon black particles in the current carbon black particle set according to the preset particle distance, until the preset number of carbon black particle sets are generated; and
constructing the percolation model of the carbon black network in the three-dimensional space based on the plurality of carbon black particle sets.

20. A computer program product, comprising a computer program, wherein the computer program, when executed by a processor, causes the processor to perform following steps:
constructing a percolation model of the carbon black network in a three-dimensional space based on model parameters, wherein the percolation model is configured to simulate a distribution of each carbon black particle within the carbon black network in the three-dimensional space;
gradually inserting new carbon black particles into the percolation model and obtaining an updated percolation model;
searching out a target conductive path among a plurality of carbon black particles included in the updated percolation model, wherein the target conductive path passes through a first surface and a second surface in the three-dimensional space, and a height difference between the first surface and the second surface in the three-dimensional space is greater than a difference threshold;
updating the model parameters, and returning to the step of constructing the percolation model of the carbon black network in the three-dimensional space based on the model parameters, until a iteration termination condition is satisfied; and
determining the percolation performance of the carbon black network based on probabilities of searching out the target conductive path in a plurality of iterations.
